# EUROPEAN PATENT SPECIFICATION

(11) **EP 2 082 752 B1**
(45) Date of publication and mention of the grant of the patent: **16.03.2011**
(21) Application number: 08001110.9
(22) Date of filing: 22.01.2008
(51) Int. Cl.: A61L 2/00, A61L 2/18, A61L 2/24

(54) **Apparatus for disinfecting medical devices**
Vorrichtung zum Desinfizieren medizinischer Geräte
Dispositif pour désinfecter des appareils médicaux

(43) Date of publication of application: 29.07.2009
(73) Proprietor: S.I.D.EM. S.p.A., 20040 Bellusco (MI) (IT)
(72) Inventor: Begni, Diego, 20066 Melezo (Milano) (IT)
(74) Representative: Lunati, Vittoriano

(56) References cited:
- EP-A- 1 709 979
- US-A- 4 710 233
- US-A- 5 882 589
- US-A1- 2007 212 278

## Description

The present invention relates to an apparatus for disinfecting medical devices of the type specified in the preamble of claim 1 and in patent application EP-A-1709979.

There are currently known a plurality of types of medical devices.

These comprise, for example, medical probes, in particular ultrasound probes.

They are placed inside the human body to extract, by means of ultrasound, images, conditions and various information regarding the internal organs, in particular the heart, lungs, liver, kidneys, etc.

Ultrasound probes are widely used in the sphere of cardiology.

In fact, viewing the heart by means of ultrasound devices placed on the outside of the human body, and in particular on the chest, is very complex due to the presence of the ribs and of other internal organs.

In said sphere of cardiology, transesophageal ultrasound probes are thus successfully utilized. These probes are inserted inside the oesophagus, and disposed in proximity of the heart to extract without impediments the images and information regarding it.

The ultrasound probes described and similar medical devices therefore come into contact with the internal organs of patients. Consequently, they must obligatorily be carefully disinfected after use.

However, these medical devices are somewhat fragile and subject to breakages. In particular, transesophageal ultrasound probes present an outer polymer sheath that prevents the electronic elements of the probe from coming into damaging contact with the saliva, fluids and internal organs of patients.

Due to this fragility it is not possible to sterilize said medical devices in an autoclave by means of high temperatures and pressures.

It is therefore necessary to chemically disinfect these medical devices by means of specific liquid disinfectants, for example by means of agents based on glutaraldehyde or peracetic acid.

However, some disinfectants can cause damage to said medical devices, and in particular to transesophageal ultrasound probes, if they come into contact with these probes for prolonged periods of time.

Moreover, some disinfectants are toxic if inhaled or if they come into contact with the human body. It is therefore necessary for these disinfectants to be totally removed from the medical devices after disinfection thereof.

It is also necessary for each washing cycle to be completed correctly, but without performing superfluous washing operations that could cause damage to the devices.

In this situation the technical aim of the present invention is to devise an apparatus for disinfecting medical devices capable of substantially overcoming the aforesaid drawbacks.

Within the scope of this technical aim, an important object of the invention is to provide an apparatus for disinfecting medical devices capable of correctly performing the disinfection processes.

Another important object of the invention is to obtain an apparatus for disinfecting medical devices capable of ensuring and monitoring correct washing in all conditions.

A further object of the invention is to devise an apparatus for disinfecting medical devices capable of ensuring correct rinsing of the medical devices.

Last but not least object of the invention is to provide an apparatus for disinfecting medical devices that verifies the integrity of the washed devices. The technical aim and objects specified are achieved by an apparatus for disinfecting medical devices described in the preamble of Claim 1. Preferred embodiments of the invention are shown by way of example in the accompanying drawings. In particular:

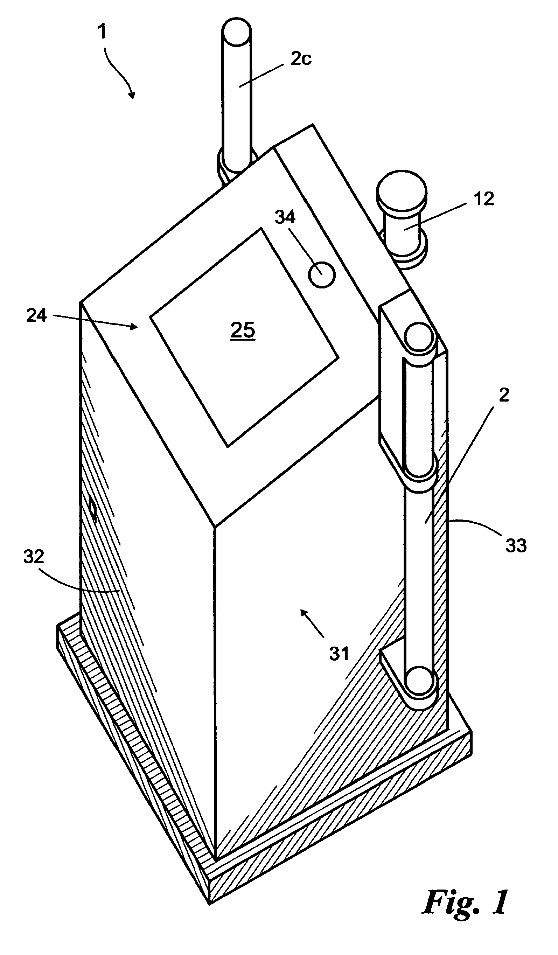

**Fig. 1** shows an external axonometric projection of an apparatus for disinfecting medical devices according to the invention;

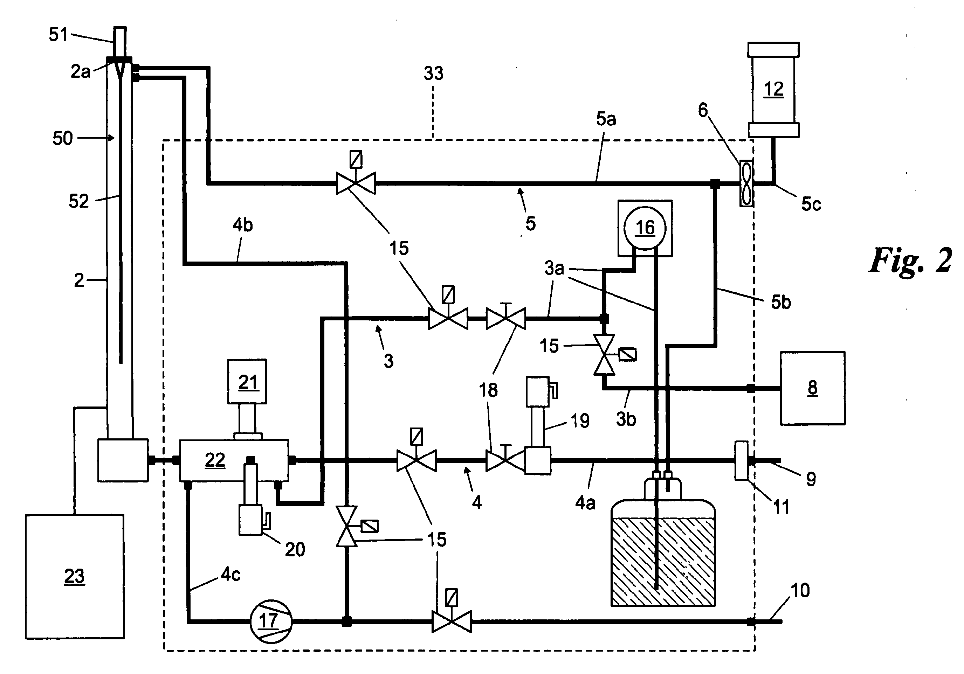

**Fig. 2** schematically shows the components of the apparatus according to the invention; and
**Fig. 3** schematizes the electrical diagram of the apparatus according to the invention.

With reference to the Figures, the disinfecting apparatus according to the invention is indicated as a whole with the number 1.

It is suitable to disinfect medical devices **50,** and in particular transesophageal ultrasound probes comprising a non-immersible part **51** and an immersible part **52** having a length in the order of size of the meter and a diameter in the order of size of the centimetre.

This immersible part 52 of said probes is suitable to be inserted in the human body and in particular into the respiratory tract, and must be disinfected before each use.

Medical devices 50, and in particular the immersible part 52 of transesophageal ultrasound probes also comprise delicate internal electrical, electronic or optical devices, subject to breakages and such that they cannot be disinfected in autoclaves or the like, but only chemically by means of specific liquid disinfectants.

More in particular, the immersible part 52 of transesophageal probes comprises an outer sheath made of polymer material suitable to insulate the internal electrical elements and to prevent the electrical current from being transferred to the patient inside whom this probe is inserted.

The apparatus 1 then comprises a housing **2** suitable to contain the medical device 50 during disinfection, and in particular the transesophageal ultrasound probe. For this purpose, the housing 2 is realized by a tube with vertical axis having a height of approximately 1.2 m and a diameter of approximately **4** cm and made of transparent polymer material. The diameter of the housing 2 is such as to allow insertion of the immersible part 52 of the transesophageal ultrasound probe, but prevent insertion of the non-immersible part 51, which does not require to be disinfected.

The housing 2 also comprises an upper opening **2a** suitable to allow rapid insertion of the medical device 50 and in particular of the immersible portion 52 of the transesophageal ultrasound probe. The upper opening 2a is also appropriately suitable to allow sealed closing of the housing 2 by means of interaction between this opening 2a and the non-immersible portion 51 of the probe.

The apparatus 1 also comprises various means for conveying fluids, and in particular first conveying means **3** of a liquid disinfectant, suitable to transfer the liquid disinfectant to the housing 2 and to empty this liquid disinfectant from the housing 2, second conveying means **4** of a rinse liquid, suitable to transfer the rinse liquid to the housing 2 and to empty this rinse liquid from the housing 2, and moreover appropriately third conveying means **5** of the aeriform substances coming from the housing 2, suitable to transfer said aeriform -substances to the outside environment.

In particular, the first conveying means 3 of the liquid disinfectant are realized by- a plurality -of first ducts **3a, flexible** and made of polymer material suitable to place a container **7** of liquid disinfectant in communication with the housing 2, and by second ducts **3b** suitable to place the housing 2 in communication with a discharge vessel **8** of the liquid disinfectant after use. Preferably, the container 7 and the discharge vessel 8 coincide, i.e. they are realized by a single element suitable to contain the liquid disinfectant before and after use. Moreover, a section of the ducts 3a and 3b can coincide.

Alternatively, the discharge vessel 8 could be replaced by specific ducts.

The second conveying means **4** of the rinse liquid are also realized by first channels **4a** suitable to place the pipes of the water supply system **9** in communication with the housing 2, and by second channels **4b** suitable to place the housing 2 in communication with discharge pipes **10** of the rinse liquid, directly in communication with a sewer system or the like. Moreover, secondary channels **4c** are preferably provided for discharge of the rinse liquid, also suitable to place the housing 2 in communication with the discharge pipes 10 and partly coinciding with the second ducts 4b. Moreover, the second conveying means 4 of the rinse liquid comprise a filter **11** suitable to filter the rinse liquid, appropriately realized substantially by water, coming from the water supply system 9.

Finally, the third conveying means 5 of the aeriform substances are realized by a plurality of first flexible pipes **5a** made of polymer material, suitable to place the upper part of the housing 2 in communication with the -outside environment and moreover by second flexible pipes **5b** made of polymer material suitable to place the container 7 of the liquid disinfectant in communication with the outside environment.

Appropriately, the pipes 5a and 5b lead into a single discharge pipe **5c** comprising a specific discharge filter **12,** suitable to retain the toxic substances and to emit purified aeriform substances into the environment. It is appropriately realized by an active carbon absorption filter.

The apparatus 1 also comprises monitoring means **13** of the conveying means 3, 4 and 5 and control means **14** suitable to control at least the motion of the liquids inside the conveying means 3, 4 and 5 and to receive information from the monitoring means 13.

More in particular, the control means 14 comprise a plurality of valves **15,** realized in particular by solenoid valves suitable to close or open the ducts on which they are positioned.

In particular, the valves 15 are present in a number of five and positioned along the ducts 3a, 3b, and the channels 4a, 4b and 4c.

The control means **14** also preferably comprise a pump **16** for the liquid disinfectant suitable to transfer the liquid along the conveying means 3 of the liquid disinfectant, and in particular along the first ducts 3a. This pump 16 is appropriately realized by a peristaltic pump, i.e. by a pump that moves the liquid by means of compression that occludes the pipes, so that the liquid disinfectant does not come into direct contact with mechanical members and the like and is not contaminated by these members. This peristaltic pump 16 preferably has a flow rate of 3 litres per minute.

The control means 14 then comprise a pump **17** of the rinse liquid suitable to transfer the rinse liquid along the conveying means **4** of the rinse liquid, and in particular along the second channels 4b. This pump 17 is appropriately realized- by a vane pump with a flow rate of 2.5 litres per minute.

Moreover, the control means 14 comprise a fan **6,** suitable to transfer the aeriform substances through the third conveying means 5 of the aeriform substances and in particular through the duct 5c.

The control means **14** also comprise flow regulators **18,** i.e. valves that open to a greater or lesser extent suitable to regulate the flow of the conveying means 3 and 4 and in particular of the ducts 3a and of the channels 4a. These flow regulators 18 can preferably be regulated by means of a manually operated tap.

The monitoring means 13 comprise a pressure gauge **19,** suitable to monitor the pressure of the conveying means 4 of the rinse liquid and in particular of the first channels **4a.**

They also preferably comprise a level meter **20,** suitable to measure the level of liquid inside the housing 2, appropriately by means of measuring the pressure exerted by this liquid at the base of the housing 2.

The monitoring means 13 also comprise a pH meter **21,** suitable to measure the pH of the liquid contained inside the housing 2.

Moreover, a manifold type hydraulic device **22** is also provided, disposed in proximity of the base of the housing 2 and in fluid communication therewith. This manifold device 22 allows connection of the housing 2 with the ducts 3a, the channels 4a and 4b, with the pH meter 21 and with the level meter 20.

The monitoring means 13 also comprises an electrical resistance meter **23** of the medical device 50, suitable to verify the integrity of this medical device 50 and in particular the presence of any lacerations on the immersible part 52 of the transesophageal ultrasound probe.

This conductivity measurement is performed by applying an electrical current with a voltage of 1000 V to the liquid in which the medical device 50 is immersed and measuring, using an Ohmmeter, the resistance between the bath and the equipotential grounding of the probe.

The apparatus 1 also comprises an electronic processor **24,** appropriately comprising a touch screen display **25,** suitable to communicate directly with the user of the apparatus 1.

This electronic processor 24 is appropriately realized by a Personal Computer including an internal memory, means for connection with a printer and with an external memory.

It is also in communication with the monitoring means 13 and suitable to regulate the control means 14.

The electronic processor 24 is in fact in connection with a plurality of relays and of various devices, of known type, suitable to control the valves 15, the pumps 16 and 17, the fan 6 and the electrical resistance meter 23 of the medical device 50. The electronic processor 24 also receives information from these controlled elements and also from the pressure gauge 19, from the level meter 29 and from the pH meter 21.

The disinfection apparatus 1 is electrically powered by the power supply network **26.**

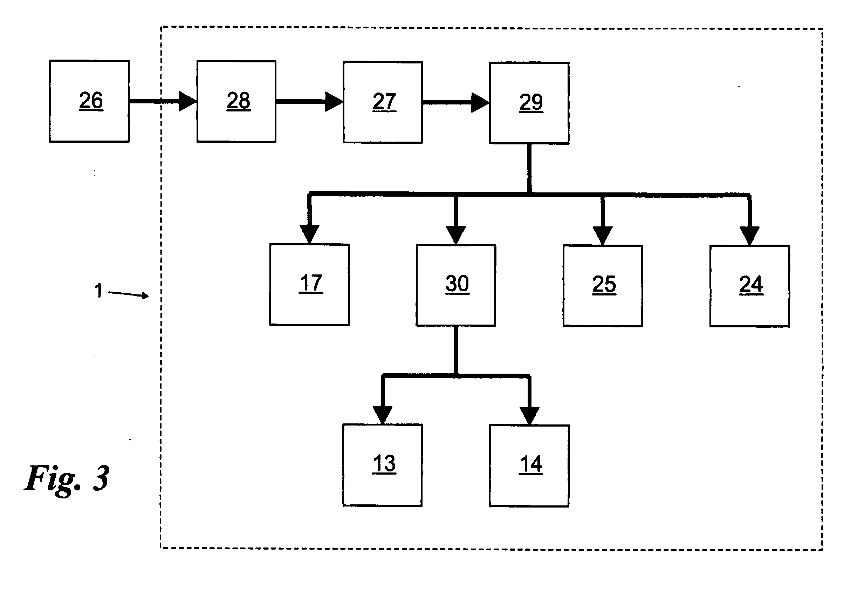

In particular, as shown in Fig. 3, the power supply network 26 is in -communication with an isolation transformer **27** suitable to isolate the apparatus 1 from any sudden voltage changes in the power supply network 26. The isolation transformer 27 can also be isolated from the power supply network 26 by means of a switch **28.**

The isolation transformer 27 is also in communication with an Uninterruptible Power Supply **29,** known with the acronym UPS, realized by a known device suitable to maintain the apparatus 1, and in particular the electronic processor 24, constantly supplied with power even in the event of a blackout of the power supply network 26.

The UPS 29 is in turn in contact with the electronic processor 24, with the display 25, with the rinse liquid pump 17 and with a direct current power supply unit **30.**

This direct current power supply unit 30 is suitable to control the control 14 and monitoring 13 means with a voltage of 24 Volt.

Structurally, the apparatus 1 comprises an external casing **31,** realized by a metal parallelepiped having the upper face including the display 25 and not parallel with the lower face. Moreover, beside the external casing 31 and on the outside thereof there is also disposed the housing 2. In the same position and on the opposite side to the housing 2, there is appropriately positioned a secondary housing **2c,** suitable to contain the medical devices 50 in conditions of non-use.

The external casing 31 comprises a front compartment **32** and a rear compartment **33.**

In particular, the front compartment 32 comprises a container 7, the isolation transformer 27, the electronic processor 24 and the UPS 29, while the remaining components are disposed in the rear compartment 33.

The disinfection apparatus 1 finally comprises wheels for transport, positioned on the lower face, and an emergency button **34,** suitable to switch off the apparatus 1 in emergency conditions.

Operation of a disinfection apparatus 1, described above in structural terms, is the following.

The apparatus 1 is activated, and therefore the switch 28 is located in an accessible position and the power supply network 26 supplies the isolation transformer 27. Moreover the UPS 29 supplies the entire apparatus 1 according to the diagram previously described and shown in Fig. 3.

In the front compartment 32 there is positioned the container 7 of liquid disinfectant and connected to the first ducts 3a and to the second pipes 5b, while the medical device 50 is positioned in the housing 2.

This liquid disinfectant can be of any type and is preferably realized by agents based on glutaraldehyde or peracetic acid.

The user can then adjust operation of the apparatus 1 by means of the electronic processor 24 and in particular of the display 25.

The disinfection cycles and the rinse cycles of medical devices 50 are prearranged.

The disinfection cycles provide for filling the housing 2 containing the medical device 50 with liquid disinfectant, leaving the liquid disinfectant and the medical device 50 inside the housing 2 for an adequate time and discharging the liquid disinfectant from the housing 2.

In particular, when the disinfection cycle is activated, the valves disposed along the first ducts 3a of the first conveying means 3 of the liquid disinfectant are opened and the pump 16 is activated to transfer the liquid disinfectant from the container 7 to the housing 2.

The level meter 20-calculates the quantity of-liquid disinfectant that is fed into the housing 2 and communicates this to the electronic processor 24, which blocks the pump 16 and the valve of the first ducts 3a when this liquid has reached an adequate level and the medical device 50 is immersed therein.

In the meantime, the container 8 always remains in connection with the second pipes 5b, part of the third conveying means 5 of the aeriform substances, so that the exhaled fumes are filtered by the discharge filter 12 and only subsequently released into the outside environment.

After the liquid disinfectant and the medical device 50 have been left inside the housing 2 for an adequate time, which can for example be approximately ten minutes if the liquid disinfectant is realized by agents based on glutaraldehyde and approximately twenty minutes if the liquid disinfectant is realized by agents based on peracetic acid, the liquid disinfectant is discharged into the discharge vessel 8.

In particular, the valves that obstruct the second ducts 3b, which as previously indicated can partly or totally coincide with the first ducts 3a, are opened and the liquid is discharged by gravitational force into the vessel 8. Alternatively, the same pump 16 in reverse direction or a specific pump can be utilized. In parallel, the valve of the first pipes 5a is also opened to allow evacuation of the fumes coming from the liquid disinfectant coming from the housing 2.

The rinse cycles instead provide for flow of the rinse liquid, substantially composed of water, inside the housing 2, containing the disinfected medical device 50 that has just been disinfected, and final discharge of this liquid.

In particular, the valves that obstruct the channels 4a and 4b of the second conveying means of the rinse liquid are opened.

The rinse liquid therefore enters from the water supply system 9 or the like, is filtered by the filter 11 and reaches the housing 2 through the first channels 4a. It therefore fills the entire housing 2 and circulates through the second channels 4b, having a connection at the top of the housing 2, to then end up in the discharge pipes 10.

The rinse cycle described utilizes the pressure provided by the water supply network 9, which is approximately 3 bar (1 bar = 0.1 MPa) and which is appropriately controlled by the pressure gauge 19.

During the rinse cycle the pH of the liquid contained in the housing 2 is monitored by the pH meter 21. In this way it is possible to verify the quantity of liquid disinfectant still present in the housing 2 and on the medical device 50.

When the pH has reached adequate values in the vicinity of 7 the rinse liquid is discharged into the discharge pipes 10, through the secondary channels 4c and by means of the pump 17.

During the rinse cycle it is also possible to check the integrity of the medical device 50 by means of the electrical resistance meter 23.

In fact, if the medical device 50 realized by the transesophageal ultrasound probe has .lacerations in the outer protective sheath, the conductivity of the rinse liquid/immersible portion of the probe system would present an electrical resistance of below 200-MOhm; while an electrical resistance of the system above this value excludes structural damage of the medical device 50.

During execution of these cycles the electronic operator 24 stores the state of the cycle and does not allow removal of the probe until the cycles provided for have terminated.

In particular, if there is an interruption in the current from the power supply network 26 or the rinse liquid from the water supply network or the like, by means of the UPS the electronic processor would store the state of the cycles in the memory, so that they can be resumed without having to be restarted from the beginning or without signalling false completion of the cycles.

Moreover, the data of the cycles are contained inside the memory of the electronic processor 24 and can be printed on paper or transferred to external memories.

The invention achieves important advantages.

In fact, the apparatus 1 is capable of correctly performing disinfection of medical devices 50 and in particular of transesophageal ultrasound probes. These processes are, in fact, always precisely monitored by the control and monitoring means 14 and 13.

Another important advantage is given by the fact that the apparatus 1 is capable of ensuring and monitoring correct washing of the devices 1 in all conditions and even in the case of interruption in the power 26 or water supply network, as previously described.

A further advantage of the invention is given by the presence of the pH meter 21, capable of verifying that the sterilized medical device 50 does not contain traces of liquid disinfectant, which could be harmful in contact with the human body.

Yet another advantage is given by the fact that the apparatus 1 does not emit toxic vapours, but filters these through the specific discharge filter 12.

Last but not least advantage is given by the presence of the electrical resistance meter 23, which allows verification of the integrity of the medical device 50 and in particular of the immersible portion of transesophageal ultrasound probes.

## Claims

1. Apparatus for disinfecting medical devices comprising a housing (2) suitable to contain at least a medical device (50) during disinfection, first conveying means (3) of a liquid disinfectant, suitable to transfer said liquid disinfectant to said housing (2) and to empty said liquid disinfectant from said housing (2), second conveying means (4) of a rinse liquid, suitable to transfer said rinse liquid to said housing (2) and to empty said rinse liquid from said housing (2), monitoring means (13) of said first and second conveying means (3, 4), suitable to verify the conditions of said liquids, control means (14) of said first and second conveying means (3, 4) suitable to control the motion of said liquids inside said first and second conveying means (3, 4), and an electronic processor (24) in communication with said monitoring means (13) and with said control means (14) and suitable to control performance of disinfection cycles and of rinse cycles and **characterized in that** said monitoring means (13) comprise an electrical resistance meter (23) of the medical device (50), suitable to verify the integrity of said medical device (50).

2. Apparatus according to claim 1, wherein said electronic processor (24) is suitable to store the conditions of said cycles.

3. Apparatus according to claim 2, wherein said electronic processor (24) is electrically connected to an uninterruptible power supply (29) connected to a power supply network (26) and suitable to allow supply of said electronic processor (24) and said storing of said cycles also in the case of interruptions in the power supply network.

4. Apparatus according to one or more of the preceding claims, wherein said monitoring means (13) comprise a pH meter (21) suitable to measure the pH of the liquid contained in said housing (2).

5. Apparatus according to one or more of the preceding claims, including third conveying means (5) of the aeriform substances coming from said housing (2) and suitable to transfer said aeriform substances to the outside environment.

6. Apparatus according to claim 5, wherein said third conveying means (5) of the aeriform substances are also suitable to transform said aeriform substances from a container (7) to said outside environment.

7. Apparatus according to claim 5 or 6, wherein said third conveying means (5) of the aeriform substances comprise a discharge filter (12) suitable to filter said aeriform substances before release into the outside environment.

8. Apparatus according to one or more of the preceding claims, wherein said housing (2) is suitable to contain a transesophageal ultrasound probe and is realized by a tube with vertical axis.

9. Apparatus according to one or more of the preceding claims, comprising an external casing (31) including a front compartment (32) suitable to include a container (7) of said liquid disinfectant.

## Patentansprüche

1. Vorrichtung zum Desinfizieren medizinischer Geräte bestehend aus einem Gehäuse (2), das in der Lage ist, mindestens ein medizinisches Gerät (50) während der Desinfektion aufzunehmen, weiters aus ersten Fördermitteln (3) einer Desinfektionsflüssigkeit, welche genannte Desinfektionsflüssigkeit in genanntes Gehäuse (2) leiten und genanntes Gehäuse (2) von genannter Desinfektionsflüssigkeit entleeren, aus zweiten Fördermitteln (4) einer Spülflüssigkeit, welche die genannte Spülflüssigkeit in genanntes Gehäuse (2) leiten und genanntes Gehäuse (2) von genannter Spülflüssigkeit entleeren, aus Kontrollmitteln (13) der genannten ersten und zweiten Fördermittel (3, 4), welche den Zustand der genannten Flüssigkeiten überprüfen und aus Antriebsmitteln (14) der genannten ersten und zweiten Fördermittel (3, 4), welche die Bewegung der genannten Flüssigkeiten im Inneren der genannten ersten und zweiten Fördermittel (3, 4) steuern und aus einer elektronischen Verarbeitungsanlage (24), die mit genannten Kontrollmitteln (13) und genannten Antriebsmitteln (14) verbunden ist und die Durchführung der Desinfektions- und der Spülzyklen steuert, **dadurch gekennzeichnet, dass** genannte Kontrollmittel (13) einen Widerstandsmesser (23) des genannten medizinischen Gerätes (50) umfassen, der die Unversehrtheit des genannten medizinischen Gerätes (50) prüft.

2. Vorrichtung nach Anspruch 1, worin genannte elektronische Verarbeitungsanlage (24) in der Lage ist, den Zustand genannter Zyklen zu speichern.

3. Vorrichtung nach Anspruch 2, worin genannte elektronische Verarbeitungsanlage (24) mit einer an ein Stromnetz (26) angeschlossenen unterbrechungsfreien Stromversorgung (29) verbunden ist, welche die Versorgung der genannten elektronischen Verarbeitungsanlage (24) und die Speicherung der genannten Zyklen auch bei Unterbrechungen am Stromnetz ermöglicht.

4. Vorrichtung nach einem oder mehreren der vorstehenden Ansprüche, worin genannte Kontrollmittel (13) einen pH-Messer (21) umfassen, der den pH-Wert der in genanntem Gehäuse (2) befindlichen Flüssigkeit misst.

5. Vorrichtung nach einem oder mehreren der vorstehenden Ansprüche, bestehend aus dritten Fördermitteln (5) der aus genanntem Gehäuse (2) kommenden gasförmigen Substanzen, welche genannte gasförmige Substanzen in die Umgebung führen.

6. Vorrichtung nach Anspruch 5, worin genannte dritte Fördermittel (5) der gasförmigen Substanzen ferner geeignet sind, die genannten gasförmigen Substanzen von genanntem Behälter (7) in genannte Außenumgebung zu führen.

7. Vorrichtung nach Anspruch 5 oder 6, worin genannte Fördermittel (5) der gasförmigen Substanzen einen Ablassfilter (12) umfassen, der die gasförmigen Substanzen vor deren Auslass in die Umgebung filtert.

8. Vorrichtung nach einem oder mehreren der vorstehenden Ansprüche, worin genanntes Gehäuse (2) in der Lage ist, eine transösophageale Echokardiographiesonde zu enthalten und aus einer Röhre mit senkrechter Achse besteht.

9. Vorrichtung nach einem oder mehreren der vorstehenden Ansprüche, bestehend aus einer Außenhülle (31), umfassend einen Vorderraum (32), der in der Lage ist, einen Behälter (7) der genannten Desinfektionsflüssigkeit zu enthalten.

## Revendications

1. Dispositif pour désinfecter des appareils médicaux comprenant un logement (2) apte à contenir au moins un dispositif médical (50) pendant la désinfection, des premiers moyens de canalisation (3) d'un liquide désinfectant, destinés à transférer ledit liquide désinfectant audit logement (2) et à vider ledit logement (2) dudit liquide désinfectant, des deuxièmes moyens de canalisation (4) d'un liquide de rinçage destinés à transférer ledit liquide de rinçage audit logement (2) et à vider ledit logement (2) dudit liquide de rinçage, des moyens de contrôle (13) desdits premiers et deuxièmes moyens de canalisation (3, 4) aptes à vérifier les conditions desdits liquides, et des moyens de commande (14) desdits premiers et deuxièmes moyens de canalisation (3, 4) aptes à commander le mouvement desdits liquides à l'intérieur desdits premiers et deuxièmes moyens de canalisation (3, 4) et un ordinateur électronique (24) en communication avec lesdits moyens de contrôle (13) et lesdits moyens de commande (14) et apte à commander l'exécution de cycles de désinfection et cycles de rinçage et **caractérisé en ce que** lesdits moyens de contrôle (13) comprennent un mesureur de résistance électrique (23) dudit appareil médical (50) destiné à vérifier l'intégrité dudit appareil médical (50).

2. Dispositif selon la revendication 1, dans lequel ledit ordinateur électronique (24) est apte à mémoriser les conditions desdits cycles.

3. Dispositif selon la revendication 2, dans lequel ledit ordinateur électronique (24) est relié électriquement à un groupe de continuité (29) relié à un réseau électrique (26) et apte à permettre l'alimentation électrique dudit ordinateur électronique (24) et la mémorisation desdits cycles même en cas d'interruptions du réseau électrique.

4. Dispositif selon une ou plusieurs des revendications précédentes, dans lequel lesdits moyens de contrôle (13) comprennent un mesureur de pH (21) destiné à mesurer le pH du liquide contenu dans ledit logement (2).

5. Dispositif selon une ou plusieurs des revendications précédentes, comprenant des troisièmes moyens de canalisation (5) des fluides aériformes en provenance dudit logement (2) et aptes à transférer lesdits fluides aériformes dans le milieu extérieur.

6. Dispositif selon la revendication 5, dans lequel lesdits troisièmes moyens de canalisation (5) des fluides aériformes sont en outre destinés à transférer lesdits fluides aériformes dudit bac (7) audit milieu extérieur.

7. Dispositif selon la revendication 5 ou 6, dans lequel lesdits troisièmes moyens de canalisation (5) des fluides aériformes comprennent un filtre d'évacuation (12) apte à filtrer lesdits fluides aériformes avant leur évacuation dans le milieu extérieur.

8. Dispositif selon une ou plusieurs des revendications précédentes, dans lequel ledit logement (2) est apte à contenir une sonde échographique transoesophagienne et se compose d'un tube à axe vertical.

9. Dispositif selon une ou plusieurs des revendications précédentes, comprenant un boîtier extérieur (31) comprenant un espace avant (32) apte à recevoir le bac (7) contenant ledit liquide désinfectant.
